# EUROPEAN PATENT SPECIFICATION

(11) **EP 4 161 360 B1**
(45) Date of publication and mention of the grant of the patent: **25.10.2023**
(21) Application number: 21732214.8
(22) Date of filing: 07.06.2021
(51) Int. Cl.: A61B 5/00, A61M 25/09

(54) **PHYSIOLOGY SENSING INTRALUMINAL DEVICE WITH REINFORCED MULTI-FILAR BUNDLE AND ASSOCIATED ASSEMBLING METHOD**
INTRALUMINALE VORRICHTUNG MIT PHYSIOLOGIEERFASSUNG UND WIEDERBELEBUNGSVERFAHREN
DISPOSITIF INTRALUMINAL DE DÉTECTION PHYSIOLOGIQUE À ENSEMBLE MULTI-FILAIRE RENFORCÉ ET PROCÉDÉ D'ASSEMBLAGE ASSOCIÉ

(30) Priority: 09.06.2020 US 202063036730 P
(43) Date of publication of application: 12.04.2023
(60) Divisional application: 23193084.3
(73) Proprietor: Philips Image Guided Therapy Corporation, San Diego CA 92130 (US); Koninklijke Philips N.V., 5656 AG Eindhoven (NL)
(72) Inventor: RICHARDSON, Mark Thomas, 5656 AE Eindhoven (NL); ABDUL, Waheed, 5656 AE Eindhoven (NL)
(74) Representative: Philips Intellectual Property & Standards
(86) International application number: PCT/EP2021/065155
(87) International publication number: WO 2021/249936

(56) References cited:
- WO-A1-2013/028737
- WO-A1-2018/095991
- US-A1- 2014 187 874
- US-A1- 2016 303 354

## Description

### TECHNICAL FIELD

The subject matter described herein relates to devices and methods for improving the strength of fine-gauge multi-filar electrical wire bundles in physiology sensing intraluminal devices. This reinforced multi-filar conductor bundle has particular but not exclusive utility for intravascular catheters and guidewires.

### BACKGROUND

The word "filar" is a noun referring to a thin thread, line, or filament. Fine electrical wires may be referred to as filars. Small-diameter medical devices such as intraluminal (e.g., intravascular) catheters and guidewires may incorporate sensors (e.g., pressure, temperature, flow, or imaging sensors) whose power and communications occur through multi-filar (e.g., bifilar, trifilar, etc.) electrical conductor bundles. Decreases in device size drive challenges in the device manufacturing process related to routing, wrapping, and securing of multi-filar electrical conductor bundles.

Current medical devices such as guidewires and catheters use a bundle of electrical filars, usually joined as two, three, or more conductive filars, to make electrical connections between electrical components, e.g. sensors or transducers and contacts. In order to make these connections, the filars may be wrapped or pulled along the length of the device, from one end of the device to the other end. Because pure copper and many copper alloys have a very low tensile and yield strength, the mechanical strength of conventional filars is low. The manufacturing process for an intraluminal catheter or guidewire device may involve wrapping or stretching of the multi-filar conductor bundle, which requires tensioning. Unfortunately, if this tensioning exceeds a threshold longitudinal force, it can cause the conductive filars to plasticly deform, which can result in undesirable elongation and/or necking, e.g., up to 100% elongation in some current processes. The associated narrowing of the conductor affects both the mechanical performance and the electrical performance of the filar, which can lead to manufacturing defects. Documents US2016303354, WO2013028737, US 2014187874 and WO2018095991 disclose relevant prior art.

The information included in this Background section of the specification, including any references cited herein and any description or discussion thereof, is included for technical reference purposes only and is not to be regarded as subject matter by which the scope of the disclosure is to be bound. The scope of the invention is defined by the claims.

### SUMMARY

Disclosed are intraluminal devices that include reinforced multi-filar conductor bundles having improved mechanical characteristics and properties. For example, improved tensile strength may reduce elongation and necking of fine-gauge filars during the manufacturing process for medical devices, thus reducing manufacturing defects. The inclusion of a reinforcing filar can reduce or eliminate plastic deformation of the multi-filar conductor bundle, while maintaining the required electrical properties.

In some presently used devices, alternate copper alloys with higher strength (e.g. beryllium copper, abbreviated as BeCu) are sometimes employed in place of pure copper in the filars of a multi-filar conductor bundle. However, these materials may have only about 15-50% of the electrical conductivity of pure copper, which may decrease the maximum current the filars can carry. The present disclosure therefore addresses this issue by retaining pure copper, high-conductivity copper alloys, or other high-conductivity materials for a majority of the filars in a multi-filar conductor bundle, but adds (or replaces one of the low-strength conducting filars with) a reinforcing filar made of a high-strength material such as stainless steel. The reinforcing filar may or may not have an insulative coating, and may or may not be a different gauge than the other filars in the bundle. Any manufacturing or assembly process that involves tensioning of the multi-filar bundle may benefit from the reinforced multi-filar conductor bundle of the present disclosure. Depending on the implementation, the reinforcing filar may be used in the electrical circuit as a conductor, or may run alongside the other electrical filars but not be electrically connected.

The reinforced multi-filar conductor bundle disclosed herein has particular, but not exclusive, utility for intraluminal medical catheters and guidewires. One general aspect of the reinforced multi-filar conductor bundle includes an intraluminal sensing guidewire. The intraluminal sensing guidewire includes a flexible elongate member configured to be positioned within a body lumen of the patient, where the flexible elongate member includes a metallic core wire extending along a longitudinal axis; a sensing element disposed at a distal portion of the flexible elongate member and configured to obtain physiological data while positioned within the body lumen, and an electrical connector disposed at a proximal portion of the flexible elongate member. The intraluminal sensing guidewire also includes a filar bundle disposed between the sensing element and the electrical connector, the filar bundle including: a first conductive filar including a first material strength, where the first conductive filar is in electrical communication with sensing element and the electrical connector; and a first reinforcement filar including a second material strength greater than the first material strength.

Implementations may include one or more of the following features. In some embodiments, the first conductive filar and the first reinforcement filar extend alongside one another in a helical manner around the metallic core wire. In some embodiments, the filar bundle further includes a second conductive filar including the first material strength, where the second conductive filar is in electrical communication with sensing element and the electrical connector. In some embodiments, the filar bundle further includes a second reinforcement filar including the second material strength. In some embodiments, the first reinforcement filar includes an insulative coating. In some embodiments, the first conductive filar includes an insulative coating. In some embodiments, the filar bundle further includes an outer coating that mechanically couples the first conductive filar to the first reinforcement filar along at least a portion of a length of the first conductive filar and the first reinforcement filar. In some embodiments, the first conductive filar includes copper. In some embodiments, the first reinforcement filar includes stainless steel. In some embodiments, the first reinforcement filar includes a cross-sectional diameter larger than a cross-sectional diameter of the first conductive filar. In some embodiments, yhe length of the first reinforcement filar is less than the length of the first conductive filar. In some embodiments, the filar bundle includes a helical section and a non-helical section, where, in the non-helical section, at least the first conductive filar extends longitudinally along a length of the flexible elongate member toward at least one electrical contact, where the first conductive filar is electrically connected to a conductive ribbon embedded within the flexible elongate member via the electrical contact.

One general aspect includes a method for assembling an intraluminal sensing guidewire. The method includes providing a flexible elongate member configured to be positioned within a body lumen of a patient, where the flexible elongate member includes a metallic core wire extending along a longitudinal axis; attaching a sensing element at a distal portion of the flexible elongate member, where the sensing element is configured to obtain the physiological data while positioned within the body lumen; attaching an electrical connector at a proximal portion of the flexible elongate member; assembling a filar bundle, where assembling a filar bundle includes coupling a conductive filar to a reinforcement filar, where the conductive filar includes a first material strength, and where the reinforcement filar includes a second material strength greater than the first material strength. The method also includes wrapping the filar bundle in a helical manner around the metallic core wire. The method also includes coupling at least the conductive filar of the filar bundle to the sensing element and the electrical connector.

Implementations may include one or more of the following features. In some embodiments, the conductive filar includes copper surrounded by an insulative coating. In some embodiments, the reinforcement filar includes stainless steel. In some embodiments, the reinforcement filar includes a cross-sectional diameter that is larger than a cross-sectional diameter of the conductive filar. In some embodiments, the reinforcement filar is shorter than the conductive filars. In some embodiments, assembling the filar bundle includes attaching the conductive filar to the reinforcement filar. Attaching the conductive filar to the reinforcement filar includes attaching an insulating coating of the conductive filar to an insulating coating of the conductive filar. In some embodiments, attaching the conductive filar to the reinforcement filar includes forming an insulating layer around the conductive filar and the reinforcement filar. In some embodiments, wrapping the filar bundle in the helical manner around the metallic core wire includes wrapping the filar bundle in the helical manner around a first section of the metallic core wire, and where the method further includes coupling the filar bundle to the flexible elongate member such that the filar bundle extends along a different second section of the metallic core wire in a linear manner.

One general aspect includes an intravascular flow-sensing guidewire. The intravascular flow-sensing guidewire also includes a flexible elongate member configured to be positioned within a blood vessel, where the flexible elongate member includes a metallic core wire extending along a longitudinal axis; a flow sensor disposed at a distal portion of the flexible elongate member and configured to sense a velocity of blood flow within the blood vessel, an electrical connector disposed at a proximal portion of the flexible elongate member. The intravascular flow-sensing guidewire also includes a filar bundle disposed between the flow sensor and the electrical connector, the filar bundle including: a first conductive filar including a first material having a first material strength; a second conductive filar including the first material, where the first conductive filar and the second conductive filar are in electrical communication with the flow sensor and the electrical connector; and a reinforcement filar including a different, second material of a second material strength greater than the first material strength, where the first conductive filar, the second conductive filar, and the reinforcement filar extend alongside one another in a helical manner around at least a portion of a length of the metallic core wire.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the reinforced multi-filar conductor bundle, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
**Figure 1** is a diagrammatic side view of an intravascular sensing system that includes an intravascular device comprising an a multi-filar electrical conductor bundle, according to aspects of the present disclosure.
**Figure 2** is a perspective view of an example electronic component of an intravascular device, in accordance with aspects of the present disclosure.
**Figure 3** is a perspective view of a multi-filar conductor bundle, in accordance with aspects of the present disclosure.
**Figure 4** is a cross-sectional view of a multi-filar conductor bundle, in accordance with at least one embodiment of the present disclosure.
**Figure 5** is a cross-sectional view of a multi-filar conductor bundle, in accordance with at least one embodiment of the present disclosure.
**Figure 6** is a cross-sectional view of a multi-filar conductor bundle, in accordance with at least one embodiment of the present disclosure.
**Figure 7** is a cross-sectional view of a multi-filar conductor bundle, in accordance with at least one embodiment of the present disclosure.
**Figure 8** is a cross-sectional view of a multi-filar conductor bundle, in accordance with at least one embodiment of the present disclosure.
**Figure 9** is a cross-sectional view of a multi-filar conductor bundle, in accordance with at least one embodiment of the present disclosure.
**Figure 10** is a diagrammatic side view of an intravascular device comprising a reinforced multi-filar electrical conductor bundle, in accordance with at least one embodiment of the present disclosure.
**Figure 11** is a is a diagrammatic side view of an intravascular device comprising a multi-filar electrical conductor bundle, in accordance with at least one embodiment of the present disclosure.
**Figure 12** is a schematic diagram of a processor circuit, in accordance with at least one embodiment of the present disclosure.
**Figure 13** is a cross-sectional view of a multi-filar conductor bundle in accordance with at least one embodiment of the present disclosure.

### DETAILED DESCRIPTION

The reinforced multi-filar conductor bundle of the present disclosure provides an improved overall material strength of a filar assembly, to reduce elongation and necking of fine-gauge filars during the manufacturing process for medical devices. The fine gauge wires may, for example, have a size of 40-54 American Wire Gauge (AWG) or a diameter of 0.0799 - 0.01575 millimeters. Improving the tensile strength of the multi-filar conductor bundle can reduce manufacturing defects in small electronic devices, such as intravascular medical catheter and guidewire devices.

The introduction of a reinforcing filar can reduce or eliminate plastic deformation of the multi-filar conductor bundle, while maintaining electrical properties and performance. This may be accomplished by including one or more higher-strength materials into one or more of the fine gauge multi-filar electrical conductor bundles to optimize their processing and assembly into electro-mechanical devices. Often these electrical conductors are incorporated into medical devices for their electrical properties only, and do not require high mechanical strength. However, in the exemplary case of medical guidewires and catheters, there are manufacturing and assembly processes that could benefit from using electrical conductor bundles (e.g., cables) with higher mechanical strength.

Aspects of the present disclosure can include one or more features described in U.S. Patent Publication Nos. 2014/0187874, 2015/0273187, 2015/0297138, 2016/0303354, 2016/0058977, and 2019/0059817, and U.S. Patent No. 9,770225.

The present disclosure therefore provides multi-filar conductor bundles that include pure copper or high-conductivity copper alloys for a majority of the filars in the bundle, and either (a) one or more non-conducting reinforcing filar with high tensile and high yield strength to the electrical conductor bundle without changing the number of conductive filars, (b) a conductive filar made of a high-strength material such as stainless steel, or (c) increasing the diameter of one of the filars, with or without changing its composition. Any of these approaches will increase the overall strength of the multi-filar bundle, while maintaining desirable electrical properties for at least a majority of the conductors. The reinforcing filar may or may not have an insulative coating and may or may not have a different diameter than the other filars in the bundle. For example, high-strength stainless steel may have a substantially lower electrical conductivity (e.g., 3-15% of the conductivity of pure copper). Including one or more lower-conductivity filars along with relatively higher-conductivity filars may be suitable for use if not all filars carry large currents or high-frequency signals (e.g., a signal ground).

The fabrication of the multi-filar conductor bundle may be performed by a supplier, based on a component specification or material specification. The reinforced multi-filar conductor bundle may advantageously improve assembly workflows of intraluminal medical devices. Depending on the implementation, the reinforcing filar may be used in the electrical circuit as a signal-carrying conductor, as a ground conductor, or as a structural reinforcement that runs alongside the other electrical filars up to the point of termination and then not used in the electrical connections.

These descriptions are provided for exemplary purposes only, and should not be considered to limit the scope of the reinforced multi-filar conductor bundle. Certain features may be added, removed, or modified without departing from the spirit of the claimed subject matter.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. Additionally, while the description below may refer to blood vessels, it will be understood that the present disclosure is not limited to such applications. For example, the devices, systems, and methods described herein may be used in any body chamber or body lumen, including an esophagus, veins, arteries, intestines, ventricles, atria, or any other body lumen and/or chamber. For the sake of brevity, however, the numerous iterations of these combinations will not be described separately.

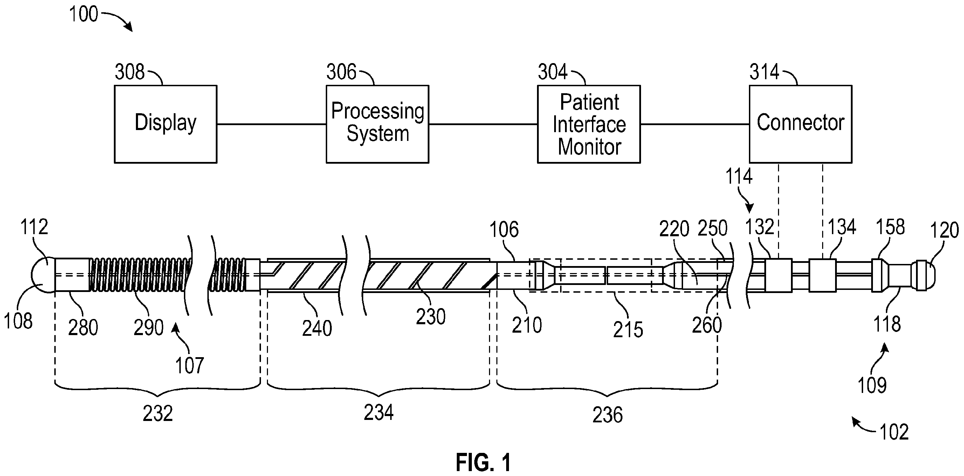

**Figure 1** is a diagrammatic side view of an intravascular sensing system 100 that includes an intravascular device 102 comprising a multi-filar electrical conductor bundle 230, according to aspects of the present disclosure. The intravascular device 102 can be an intravascular guidewire sized and shaped for positioning within a vessel of a patient. The intravascular device 102 can include a distal tip 108 and a sensing component 112. The sensing component 112 can be an electronic, electromechanical, mechanical, optical, and/or other suitable type of sensor. For example, the electronic component 112 can be a flow sensor configured to measure the velocity of blood flow within a blood vessel of a patient, a pressure sensor configured to measure a pressure of blood flowing within the vessel, or another type of sensor including but not limited to a temperature or imaging sensor. For example, flow data obtained by a flow sensor can be used to calculate physiological variables such as coronary flow reserve (CFR). Pressure data obtained by a pressure sensor may for example be used to calculate a physiological pressure ratio (e.g., FFR, iFR, Pd/Pa, or any other suitable pressure ratio). An imaging sensor may include an intravascular ultrasound (IVUS), intracardiac echocardiography (ICE), optical coherence tomography (OCT), or intravascular photoacoustic (IVPA) imaging sensor. For example, the imaging sensor can include one or more ultrasound transducer elements, including an array of ultrasound transducer elements.

The intravascular device 102 includes a flexible elongate member 106. The electronic component 112 is disposed at the distal portion 107 of the flexible elongate member 106. The electronic component 112 can be mounted at the distal portion 107 within a housing 280 in some embodiments. A flexible tip coil 290 extends distally from the housing 280 at the distal portion 107 of the flexible elongate member 106. A connection portion 114 located at a proximal end of the flexible elongate member 106 includes conductive portions 132, 134. In some embodiments, the conductive portions 132, 134 can be conductive ink that is printed and/or deposited around the connection portion 114 of the flexible elongate member 106. In some embodiments, the conductive portions 132, 134 are conductive, metallic rings that are positioned around the flexible elongate member. A locking section is formed by collar 118 and knob 120 are disposed at the proximal portion 109 of the flexible elongate member 106.

The intravascular device 102 in Figure 1 includes a distal core wire 210 and a proximal core wire 220. The distal core 210 and the proximal core 220 are metallic components forming part of the body of the intravascular device 102. For example, the distal core 210 and the proximal core 220 are flexible metallic rods that provide structure for the flexible elongate member 106. The diameter of the distal core 210 and the proximal core 220 can vary along its length. A joint between the distal core 210 and proximal core 220 is surrounded and contained by a hypotube 215.

In some embodiments, the intravascular device 102 comprises a distal assembly and a proximal assembly that are electrically and mechanically joined together, which provides for electrical communication between the electronic component 112 and the conductive portions 132, 134. For example, flow data obtained by the electronic component 112 (in this example, electronic component 112 is a flow sensor) can be transmitted to the conductive portions 132, 134. Control signals (e.g., operating voltage, start/stop commands, etc.) from a processor system 306 in communication with the intravascular device 102 can be transmitted to the electronic component 112 via a connector 314 that is attached to the conductive portions 132, 134. The distal subassembly can include the distal core 210. The distal subassembly can also include the electronic component 112, the multi-filar conductor bundle 230, and/or one or more layers of insulative polymer/plastic 240 surrounding the conductive members 230 and the core 210. For example, the polymer/plastic layer(s) can insulate and protect the conductive members of the multi-filar cable or conductor bundle 230. The proximal subassembly can include the proximal core 220. The proximal subassembly can also include one or more layers of polymer layer(s) 250 (hereinafter polymer layer 250) surrounding the proximal core 220 and/or conductive ribbons 260 embedded within the one or more insulative and/or protective polymer layer(s) 250. In some embodiments, the proximal subassembly and the distal subassembly can be separately manufactured. During the assembly process for the intravascular device 102, the proximal subassembly and the distal subassembly can be electrically and mechanically joined together. As used herein, flexible elongate member can refer to one or more components along the entire length of the intravascular device 102, one or more components of the proximal subassembly (e.g., including the proximal core 220, etc.), and/or one or more components the distal subassembly 210 (e.g., including the distal core 210, etc.). The joint between the proximal core 220 and distal core 210 is surrounded by the hypotube 215.

In various embodiments, the intravascular device 102 can include one, two, three, or more core wires extending along its length. For example, in one embodiment, a single core wire extends substantially along the entire length of the flexible elongate member 106. In such embodiments, a locking section 118 and a section 120 can be integrally formed at the proximal portion of the single core wire. The electronic component 112 can be secured at the distal portion of the single core wire. In other embodiments, such as the embodiment illustrated in Figure 1, the locking section 118 and the section 120 can be integrally formed at the proximal portion of the proximal core 220. The electronic component 112 can be secured at the distal portion of the distal core 210. The intravascular device 102 includes one or more conductive members in a multi-filar conductor bundle 230 in communication with the electronic component 112. For example, the conductor bundle 230 can include one or more electrical wires that are directly in communication with the electronic component 112. In some instances, the conductive members 230 are electrically and mechanically coupled to the electronic component 112 by, e.g., soldering. In some instances, the conductor bundle 230 comprises two or three electrical wires (e.g., a bifilar cable or a trifilar cable). An individual electrical wire can include a bare metallic conductor, or a metallic conductor surrounded by one or more insulating layers. The multi-filar conductor bundle 230 can extend along a length of the distal core 210. For example, at least a portion of the conductive members 230 can be helically, or spirally, wrapped around an entire length of the distal core 210, or a portion of the length of the distal core 210.

The intravascular device 102 includes one or more conductive ribbons 260 at the proximal portion of the flexible elongate member 106. The conductive ribbons 260 are embedded within polymer layer(s) 250. The conductive ribbons 260 are directly in communication with the conductive portions 132 and/or 134. In some instances, the multi-filar conductor bundle 230 is electrically and mechanically coupled to the electronic component 112 by, e.g., soldering. In some instances, the conductive portions 132 and/or 134 comprise conductive ink (e.g., metallic nano-ink, such as silver or gold nano-ink) that is deposited or printed directed over the conductive ribbons 260.

As described herein, electrical communication between the conductive members 230 and the conductive ribbons 260 can be established at the connection portion 114 of the flexible elongate member 106. By establishing electrical communication between the conductor bundle 230 and the conductive ribbons 260, the conductive portions 132, 134 can be in electrically communication with the electronic component 112.

In some embodiments represented by Figure 1, intravascular device 102 includes a locking section 118 and a section 120. To form locking section 118, a machining process is necessary to remove polymer layer 250 and conductive ribbons 260 in locking section 118 and to shape proximal core 220 in locking section 118 to the desired shape. As shown in Figure 1, locking section 118 includes a reduced diameter while section 120 has a diameter substantially similar to that of proximal core 220 in the connection portion 114. In some instances, because the machining process removes conductive ribbons in locking section 118, proximal ends of the conductive ribbons 260 would be exposed to moisture and/or liquids, such as blood, saline solutions, disinfectants, and/or enzyme cleaner solutions, an insulation layer 158 is formed over the proximal end portion of the connection portion 114 to insulate the exposed conductive ribbons.

In some embodiments, a connector 314 provides electrical connectivity between the conductive portions 132, 134 and a patient interface module or monitor 304. The patient interface module (PIM) 304 may in some cases connect to a console or processing system 306, which includes or is in communication with a display 308. In some embodiments, the patient interface module 304 includes signal processing circuitry, such as an analog-to-digital converter (ADC), analog and/or digital filters, signal conditioning circuitry, and any other suitable signal processing circuitry for processing the signals provided by the electronic component 112 for use by the processing system 306.

The system 100 may be deployed in a catheterization laboratory having a control room. The processing system 306 may be located in the control room. Optionally, the processing system 306 may be located elsewhere, such as in the catheterization laboratory itself. The catheterization laboratory may include a sterile field while its associated control room may or may not be sterile depending on the procedure to be performed and/or on the health care facility. In some embodiments, device 102 may be controlled from a remote location such as the control room, such than an operator is not required to be in close proximity to the patient.

The intraluminal device 102, PIM 304, and display 308 may be communicatively coupled directly or indirectly to the processing system 306. These elements may be communicatively coupled to the medical processing system 306 via a wired connection such as a standard copper multi-filar conductor bundle 230. The processing system 306 may be communicatively coupled to one or more data networks, e.g., a TCP/IP-based local area network (LAN). In other embodiments, different protocols may be utilized such as Synchronous Optical Networking (SONET). In some cases, the processing system 306 may be communicatively coupled to a wide area network (WAN).

The PIM 304 transfers the received signals to the processing system 306 where the information is processed and displayed on the display 308. The console or processing system 306 can include a processor and a memory. The processing system 306 may be operable to facilitate the features of the intravascular sensing system 100 described herein. For example, the processor can execute computer readable instructions stored on the non-transitory tangible computer readable medium.

The PIM 304 facilitates communication of signals between the processing system 306 and the intraluminal device 102. In some embodiments, the PIM 304 performs preliminary processing of data prior to relaying the data to the processing system 306. In examples of such embodiments, the PIM 304 performs amplification, filtering, and/or aggregating of the data. In an embodiment, the PIM 304 also supplies high- and low-voltage DC power to support operation of the intraluminal device 102 via the multi-filar conductor bundle 230.

The multi-filar cable or transmission line bundle 230 can include a plurality of conductors, including one, two, three, four, five, six, seven, or more conductors. The multi-filar conductor bundle 230 can be positioned along the exterior of the distal core 210. The multi-filar conductor bundle 230 and the distal core 210 can be overcoated with an insulative and/or protective polymer 240. In the example shown in Figure 1, the multi-filar conductor bundle 230 includes two straight portions 232 and 236, where the multi-filar conductor bundle 230 extends linearly and parallel to a longitudinal axis of the flexible elongate member 106 on the exterior of the distal core 210, and a helical or spiral portion 234, where the multi-filar conductor bundle 230 is wrapped around the exterior of the distal core 210. In some embodiments, the multi-filar conductor bundle 230 only includes a straight portion or only includes a helical or spiral portion. In general, the multi-filar conductor bundle 230 can extend in a linear, wrapped, non-linear, or non-wrapped manner, or any combination thererof. Communication, if any, along the multi-filar conductor bundle 230 may be through numerous methods or protocols, including serial, parallel, and otherwise, wherein one or more filars of the bundle 230 carry signals. One or more filars of the multi-filar conductor bundle 230 may also carry direct current (DC) power, alternating current (AC) power, or serve as an electrical ground connection.

The display or monitor 308 may be a display device such as a computer monitor, a touch-screen display, a television screen, or any other suitable type of display. The monitor 308 may be used to display selectable prompts, instructions, and visualizations of imaging data to a user. In some embodiments, the monitor 308 may be used to provide a procedure-specific workflow to a user to complete an intraluminal imaging procedure.

Before continuing, it should be noted that the examples described above are provided for purposes of illustration, and are not intended to be limiting. Other devices and/or device configurations may be utilized to carry out the operations described herein.

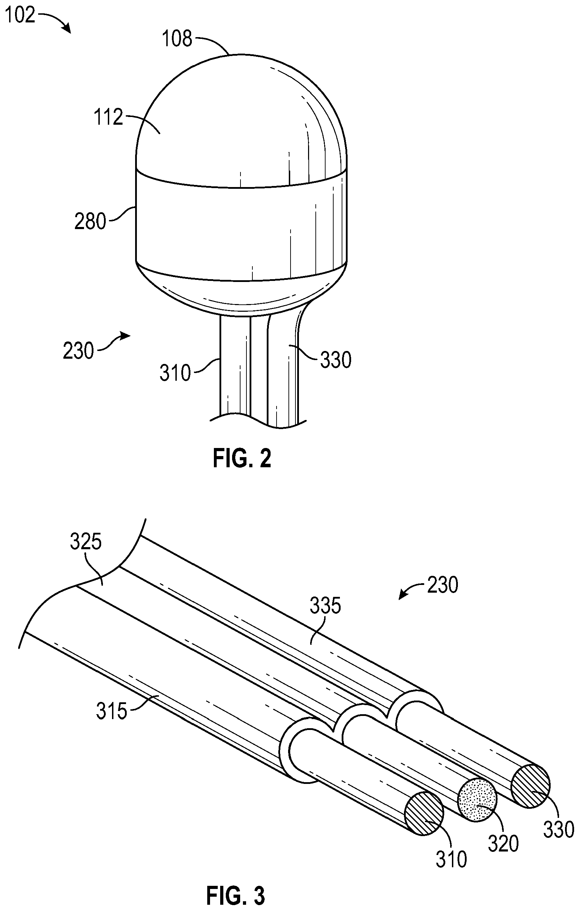

**Figure 2** is a side view of an example electronic component 112 of an intravascular device 102 in accordance with aspects of the present disclosure. For example, the electronic component 112 can be a pressure sensor, flow sensor, temperature sensor, or other sensor configured to measure a parameter of blood flow within a vessel of a patient. In an exemplary embodiment, the flow sensor is a single ultrasound transducer element. The transducer element emits ultrasound signals and receives ultrasound echoes reflected from anatomy (e.g., flowing fluid, such as blood). The transducer element generates electrical signals representative of the echoes. The signal-carrying filars carry this electrical signal from the sensor at the distal portion to the connector at the proximal portion. The processing system processes the electrical signals to extract the flow velocity of the fluid. In other embodiments, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices. In some embodiments, the electronic component 112 may include an imaging component (e.g., an intravascular ultrasound imaging component), a measurement component (e.g., a pressure, flow, or temperature sensor) and/or a treatment component (e.g., an ablation component). In some embodiments the electronic component 112 may be fully or partially enclosed within a housing 280. In some embodiments, the electronic component is located at or near the distal end of a flexible elongate member, and may include a distal tip 108 (e.g., an atraumatic tip). In some embodiments, one or more electronic components can be located at the distal portion of the flexible elongate member. For example, the one or more electronic components can be located at the distal tip (a leading edge of the flexible elongate member and/or where the distal portion terminates) or proximally spaced from the distal end (by, e.g., 0.5 cm, 1 cm, 1.5 cm, 2 cm, 3 cm, 4 cm, 5 cm, and/or other suitable values both larger and smaller). Some embodiments of the intraluminal device 102 include multiple, different electronic components (e.g., a pressure sensor and a flow sensor, or any other quantity or combination of sensors). In such embodiments, a first electronic component can be positioned at the distal tip of the flexible elongate member and the second electronic component can be spaced from the distal tip and/or from the first electronic component (by, e.g., 0.5 cm, 1 cm, 1.5 cm, 2 cm, 3 cm, 4 cm, 5 cm, and/or other suitable values both larger and smaller). In some embodiments, power, control signals, and electrical ground or signal return may be provided by the multi-filar conductor bundle 230, which in the example of Figure 2 is shown as a bifilar with two conductive filars 310 and 330. The conductive filars 310 and 330 may for example be made of pure copper, or of a copper alloy such as BeCu or AgCu.

**Figure 3** is a perspective view of a multi-filar conductor bundle 230 in accordance with aspects of the present disclosure. In the example shown in Figure 3, the multi-filar conductor bundle 230 is a trifilar that includes three conductors 310, 320, and 330, surrounded by insulating sheaths 315, 325, and 335, respectively. The insulating sheaths may for example be made of polyimide. The additional overcoating 340 may for example be applied through dip coating, although other methods may be used instead or in addition. In a conventional trifilar, all three conductors may be of the same or similar diameter, and may be made of pure copper, or of a copper alloy such as BeCu. Although the multi-filar conductor bundle 230 is shown here with the conductors arranged side-by-side in a planar fashion, a person of ordinary skill in the art will appreciate that other arrangements may be employed instead or in addition.

In the illustrated embodiment, the second filar 320 comprises a different material than the first and third filars 310, 330. In this regard, the second filar 320 may comprise a material with a relatively higher strength than the filars 310, 330. For example, the second filar 320 may comprise stainless steel or a high-strength copper alloy, while the conductive filars 310, 330 comprise other copper alloys or pure copper.

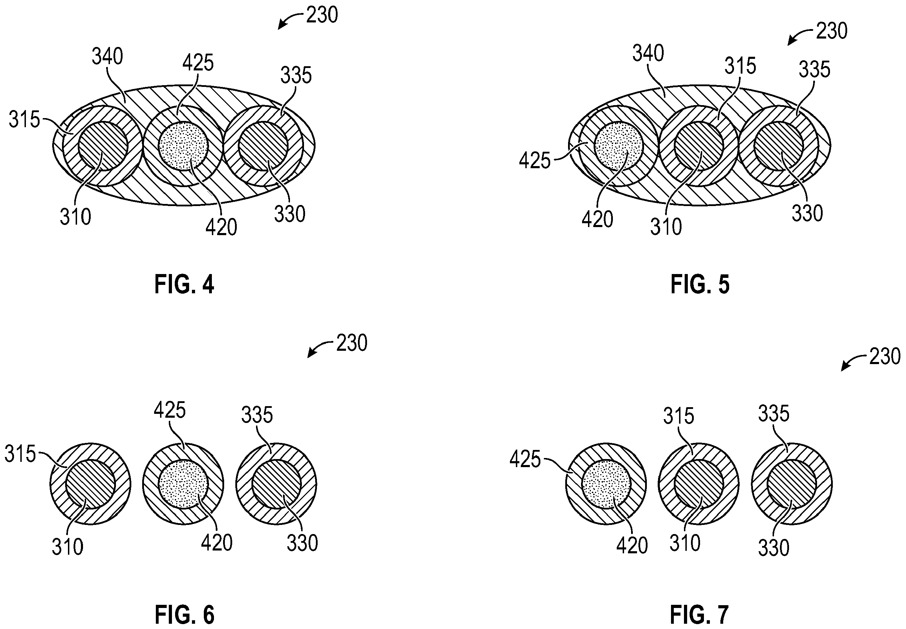

**Figure 4** is a cross-sectional view of a multi-filar conductor bundle 230 in accordance with at least one embodiment of the present disclosure. In the example shown in Figure 4, the multi-filar conductor bundle 230 is a trifilar that includes two conductive filars 310 and 330, surrounded by insulating sheaths 315 and 335, respectively. In the example shown in Figure 4, the center filar of the bundle 230 is a reinforcement filar 420, which may for example be of a material (e.g., stainless steel) that has a higher tensile strength and/or higher yield strength than the conductive material (e.g., copper or BeCu) of the conductive filars 310 and 330. The reinforcement filar 420 may include an insulating sheath 425. In other embodiments, the reinforcement filar 420 does not include an insulating sheath 425. The multi-filar conductor bundle 230 may or may not include an additional overcoating 340, such as nylon or polyurethane, that joins the separate insulators 315, 325, and 335 together such that the filars 310, 320, and 330 form a single joined conductor bundle 230. In some embodiments, the conductive filars 310 and 330, but not the reinforcement filar, are surrounded by insulating sheaths 315, 335, and the multi-filar conductor bundle 230 is covered with the overcoating 340.

The addition of a reinforcing filar 420 with a high tensile or high yield strength may increase the overall tensile strength of the multi-filar fine wire bundle 230, while maintaining desirable electrical properties for the conductive filars 310 and 330. This may for example permit pure copper to be used for the conductive filars 310 and 330 in place of copper alloys such as BeCu or AgCu, without compromising the mechanical strength of the multi-filar conductor bundle 230, and with greatly reduced risk of elongation and necking that can break filars or limit their current-carrying capacity.

In some embodiments, the reinforcing filar 420 is not connected to electrical terminals at one or both ends of the multi-filar conductor bundle. High-strength stainless steel may have a substantially lower electrical conductivity (e.g., 3-15% of the conductivity of pure copper), making it less desirable for use as an electrical conductor. However, in other instances, the reinforcing filar 430 may be employed as a conductor for certain applications. For example, in a filar that does not need to carry large currents or high-frequency signals (e.g., a signal ground wire), the lower conductivity of a reinforcing material (e.g., stainless steel) may be acceptable.

Other materials, whether conductive or not, may be employed instead of or in addition to stainless steel. In the example shown in Figure 4, the multi-filar conductor bundle 230 is shown with three filars arranged side-by-side in a planar fashion. However, other arrangements and other numbers of filars, whether conductive or reinforcing, may be employed instead or in addition. Furthermore, while the filars are shown as being circular in cross-section, some or all filars may be oval, rectangular, or curved in cross-section so as to minimize the profile of the multi-filar conductor bundle against a guidewire, catheter, or other device.

In some embodiments, the insulating sheaths 315, 335, 425 can be omitted. In such embodiments, the overcoating 340 can provide electrical isolation between the conductive filar 310, the conductive filar 330, and/or the reinforcing filar 420.

**Figure 5** is a cross-sectional view of a multi-filar conductor bundle 230 in accordance with at least one embodiment of the present disclosure. In the example shown in Figure 5, the multi-filar conductor bundle 230 is a trifilar that includes two conductive filars 310 and 330, surrounded by insulating sheaths 315 and 335, respectively. In the example shown in Figure 5, the left outer filar of the bundle 230 is a reinforcement filar 420 that may or may not include an insulating sheath 425. The reinforcement filar 420 can be located in any suitable position (e.g., the left position, the right position, the outer position, the central position, etc.). The multi-filar conductor bundle 230 may or may not include an additional overcoating 340 that joins the filars together to form a single conductor bundle 230. In the example shown in Figure 5, the multi-filar conductor bundle 230 is shown with three filars arranged side-by-side in a planar fashion. However, other arrangements and other numbers of filars, whether conductive or reinforcing, may be employed instead or in addition.

**Figure 6** is a cross-sectional view of a multi-filar conductor bundle 230 in accordance with at least one embodiment of the present disclosure. In the example shown in Figure 6, the multi-filar conductor bundle 230 is a trifilar that includes two conductive filars 310 and 330, surrounded by insulating sheaths 315 and 335, respectively. In the example shown in Figure 7, the center filar of the bundle 230 is a reinforcement filar 420 that may or may not include an insulating sheath 425. In this example, the multi-filar conductor bundle 230 does not include an additional overcoating 340 that joins the filars together. However, the filars 310, 330, and 420 may be tied, braided, or otherwise coupled to one another, or may simply be routed in a parallel but separate manner, wherein the reinforcement filar 420 helps to prevent the conductive filars 310 and 330 from elongating or necking during tensioning of the filars. In the example shown in Figure 6, the multi-filar conductor bundle 230 is shown with three filars arranged side-by-side in a planar fashion. However, other arrangements and other numbers of filars, whether conductive or reinforcing, may be employed instead or in addition.

**Figure 7** is a cross-sectional view of a multi-filar conductor bundle 230 in accordance with at least one embodiment of the present disclosure. In the example shown in Figure 5, the multi-filar conductor bundle 230 is a trifilar that includes two conductive filars 310 and 330, surrounded by insulating sheaths 315 and 335, respectively. In the example shown in Figure 7, the left outer filar of the bundle 230 is a reinforcement filar 420 that may or may not include an insulating sheath 425. In this example, the multi-filar conductor bundle 230 does not include an additional overcoating 340 that joins the filars together. However, the filars 310, 330, and 420 may be otherwise coupled to one another, or routed in a parallel but separate manner, wherein the reinforcement filar 420 helps to prevent the conductive filars 310 and 330 from elongating or necking. In the example shown in Figure 7, the multi-filar conductor bundle 230 is shown with three filars arranged side-by-side in a planar fashion. However, other arrangements and other numbers of filars, whether conductive or reinforcing, may be employed instead or in addition.

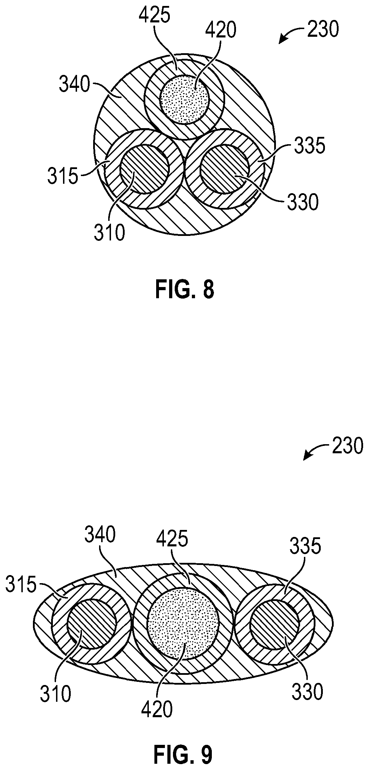

**Figure 8** is a cross-sectional view of a multi-filar conductor bundle 230 in accordance with at least one embodiment of the present disclosure. In the example shown in Figure 8, the multi-filar conductor bundle 230 is a trifilar that includes two conductive filars 310 and 330, surrounded by insulating sheaths 315 and 335, respectively. In the example shown in Figure 5, the one filar of the bundle 230 is a reinforcement filar 420 that may or may not include an insulating sheath 425. The multi-filar conductor bundle 230 may or may not include an additional overcoating 340 that joins the filars together to form a single joined conductor bundle 230. In the example shown in Figure 5, the multi-filar conductor bundle 230 is shown with three filars arranged in a triangular fashion. However, other arrangements and other numbers of filars, whether conductive or reinforcing, may be employed instead or in addition. Although a flat (side-by-side) arrangement of conductors may minimize the profile of the conductor bundle against a catheter, guidewire, or other device, a stacked (e.g., triangular, rectangular, etc.) arrangement may be advantageous or desirable for other reasons, depending on the implementation.

**Figure 9** is a cross-sectional view of a multi-filar conductor bundle 230 in accordance with at least one embodiment of the present disclosure. In the example shown in Figure 9, the multi-filar conductor bundle 230 is a trifilar that includes two conductive filars 310 and 330, surrounded by insulating sheaths 315 and 335, respectively. In the example shown in Figure 9, the center filar of the bundle 230 is a reinforcement filar 420 with a larger diameter than the conductive filars 310 and 330. In the illustrated embodiment, the reinforcement filar 420 comprises a different material with a relatively higher strength than the materials used for the conductive filars 310, 330 and is surrounded by an insulating sheath 425. In other embodiments, the reinforcement filar 420 is made of the same material as the conductive filars 310 and 330. In some embodiments, the reinforcement filar 420 does not include the insulating sheath 425. The multi-filar conductor bundle 230 may or may not include an additional overcoating 340 that joins the filars together to form a single conductor bundle 230.

The tensile strength or yield strength of a filar may be dependent on its composition. The total force required to elongate, neck, or otherwise deform a filar may be dependent on the diameter of the filar, and therefore the addition of a reinforcing filar 420, with a larger diameter than that of the conductive filars 310 and 330, may increase the total force on the multi-filar fine wire bundle 230 that is required to elongate or neck the conductive filars of the multi-filar fine wire bundle 230. In the example shown in Figure 9, the multi-filar conductor bundle 230 is shown with three filars arranged side-by-side in a planar fashion. However, other arrangements and other numbers of filars, whether conductive or reinforcing, may be employed instead or in addition. Although a flat (side-by-side) arrangement of same-size conductors may minimize the profile of the conductor bundle against a catheter, guidewire, having one or more conductors of larger diameter may be advantageous or desirable for other reasons, depending on the implementation.

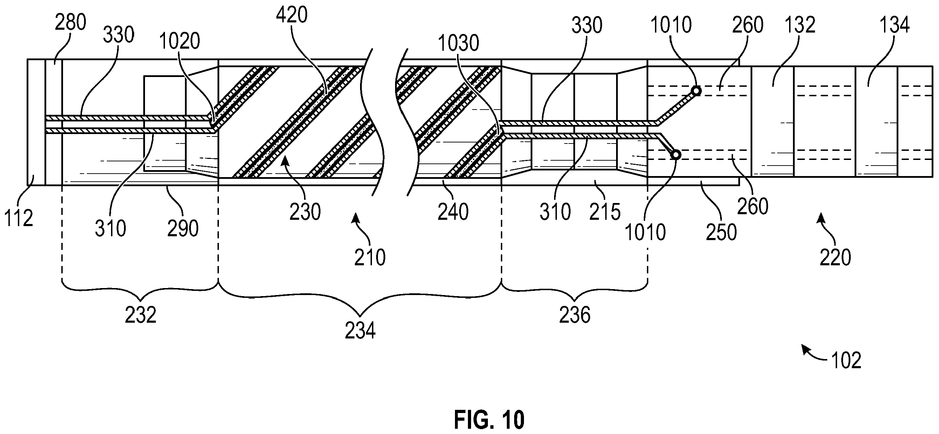

**Figure 10** is a diagrammatic side view of an intravascular device 102 comprising a reinforced multi-filar electrical conductor bundle 230, in accordance with at least one embodiment of the present disclosure. Visible are the proximal core wire 220 and distal core wire 210, joined by a hypotube 215. At the distal end of the distal core wire is a coil 290 that terminates with an electronic device 112 that may be fully or partially enclosed within a housing 280. Also visible is a reinforced multi-filar conductor bundle 230 that includes conductive filars 310 and 330, and a reinforcement filar 420.

The conductive filars 310 and 330 connect the electronic component 112 with electrical contacts 1010 formed on the conductive ribbons 260 that make electrical contact with the conductive regions 132 and 134. The reinforced multi-filar conductor bundle 230 includes a straight region 232 that passes through the coil 290. The reinforced multi-filar conductor bundle 230 also includes a helical region 234 that wraps around the distal core wire 210 and is overcoated with an insulative or protective polymer coating 240. In the helical region 234, the filars of 310, 320, 330 of the bundle extend in parallel around the distal core wire 210 such that the helical shape formed by the filars 310, 320, 330 is concentric with the distal core wire 210. The filars 310, 320, 330 are shown extending in contact with one another. In this regard, the filars 310, 320, 330 may be attached to one another, or unattached to one another such that the filars 310, 320, 330 extend adjacent to one another. The reinforced multi-filar conductor bundle 230 additionally includes a straight region 236 that passes through the hypotube 215. Between the hypotube 215 and the electrical contacts 1010, the conductive filars are overcoated with a polymer coating 250. In some embodiments, the electrical contacts 1010 are formed by removing (e.g., etching, ablating) a portion of an outer insulating coating or layer covering the ribbons 260, and soldering the conductive filars 310, 330 to the ribbons 260.

In the example shown in Figure 10, the reinforcement filar 420 is present within the multi-filar conductor bundle 230 in the helical region 234, but not in the straight regions 232 and 236. A distal end of the reinforcement filar 420 ends at a location 1020. Location 1020 is proximal of a distal end of the distal core wire 210. Location 1020 can be aligned for example with a location where a tapering at a distal portion of the distal core wire 210 begins. A proximal end of the reinforcement filar 420 ends at a location 1030. Location 1030 is distal of a proximal end of the distal core wire 210. Location 1030 can be aligned for example with a location where a tapering at a proximal portion of the distal core wire 210 begins. The reinforcement filar may for example be clipped, skived, or bent at locations 1020 and 1030 such that it is separated from the conductive filars 310 and 330 at these locations, such that it provides strength to the multi-filar conductor bundle 230 in processing-intensive regions such as the helical region 234, but does not interfere with fine assembly operations at the electrically terminated ends, and does not add unwanted stiffness to the coil 290. The multi-filar conductor bundle 230 may also be easier to feed through certain couplings with the reinforcement filar 420 removed.

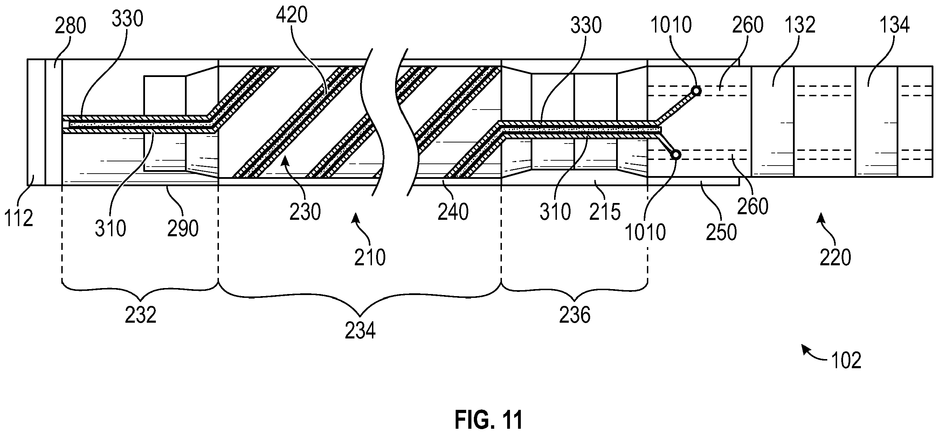

**Figure 11** is a is a diagrammatic side view of an intravascular device 102 comprising a multi-filar electrical conductor bundle 230, in accordance with at least one embodiment of the present disclosure. Visible are the proximal core wire 220 and distal core wire 210, joined by a hypotube 215. At the distal end of the distal core wire is a coil 290 that terminates with an electronic device 112 that is fully or partially enclosed in a housing 280. Also visible is a reinforced multi-filar conductor bundle 230 that includes conductive filars 310 and 330, and a reinforcement filar 420.

The conductive filars 310 and 330 connect the electronic component 112 with electrical contacts 1010 formed on the conductive ribbons 260 that make electrical contact with the conductive regions 132 and 134. The reinforced multi-filar conductor bundle 230 includes a straight region 232 that passes through the coil 290. The reinforced multi-filar conductor bundle 230 also includes a helical region 234 that wraps around the distal core wire 210 and is overcoated with a polymer coating 240. The reinforced multi-filar conductor bundle 230 additionally includes a straight region 236 that passes through the hypotube 215. Between the hypotube 215 and the electrical contacts 1010, the conductive filars are overcoated with a polymer coating 250.

In the example shown in Figure 11, the reinforcement filar 420 is present in the helical region 234, and also in the straight regions 232 and 236, but is not electrically terminated at either end. In other embodiments, the reinforcement filar 420 may be employed as a conductor, and may be electrically terminated for example at the electronic device on its distal end, and at an electrical connection 1010 at its proximal end.

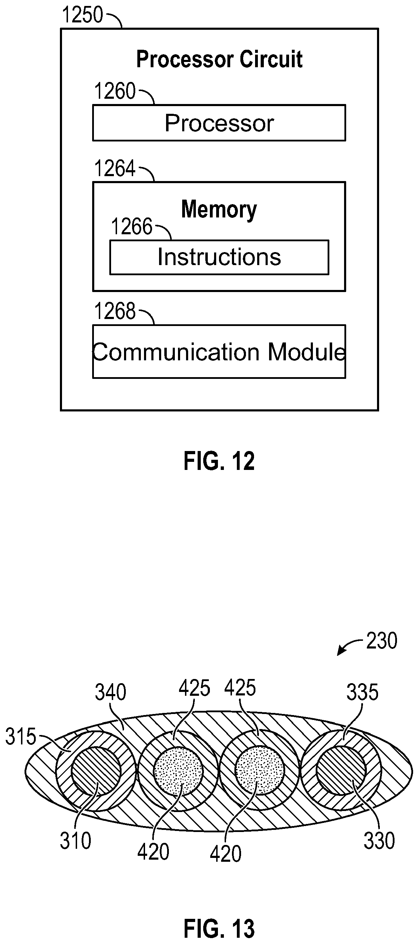

**Figure 12** is a schematic diagram of a processor circuit 1250, according to at least one embodiment of the present disclosure. The processor circuit 1250 may be implemented in the intravascular sensing system 100, or other devices or workstations (e.g., third-party workstations, network routers, etc.), or on a cloud processor or other remote processing unit, as necessary to implement the method. As shown, the processor circuit 1250 may include a processor 1260, a memory 1264, and a communication module 1268. These elements may be in direct or indirect communication with each other, for example via one or more buses.

The processor 1260 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1260 may also comprise another hardware device, a firmware device, or any combination thereof configured to perform the operations described herein. The processor 1260 may also be implemented as a combination of computing devices, e.g., a combination of a DSP and a microprocessor, a plurality of microprocessors, one or more microprocessors in conjunction with a DSP core, or any other such configuration.

The memory 1264 may include a cache memory (e.g., a cache memory of the processor 1260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1264 includes a non-transitory computer-readable medium. The memory 1264 may store instructions 1266. The instructions 1266 may include instructions that, when executed by the processor 1260, cause the processor 1260 to perform the operations described herein. Instructions 1266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1268 can include any electronic circuitry and/or logic circuitry to facilitate direct or indirect communication of data between the processor circuit 1250, and other processors or devices. In that regard, the communication module 1268 can be an input/output (I/O) device. In some instances, the communication module 1268 facilitates direct or indirect communication between various elements of the processor circuit 1250 and/or the intravascular measurement system 100. The communication module 1268 may communicate within the processor circuit 1250 through numerous methods or protocols. Serial communication protocols may include but are not limited to US SPI, I²C, RS-232, RS-485, CAN, Ethernet, ARINC 429, MODBUS, MIL-STD-1553, or any other suitable method or protocol. Parallel protocols include but are not limited to ISA, ATA, SCSI, PCI, IEEE-488, IEEE-1284, and other suitable protocols. Where appropriate, serial and parallel communications may be bridged by a UART, USART, or other appropriate subsystem.

External communication (including but not limited to software updates, firmware updates, preset sharing between the processor and central server, or readings from the ultrasound device) may be accomplished using any suitable wireless or wired communication technology, such as a cable interface such as a USB, micro USB, Lightning, or FireWire interface, Bluetooth, Wi-Fi, ZigBee, Li-Fi, or cellular data connections such as 2G/GSM, 3G/UMTS, 4G/LTE/WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

**Figure 13** is a cross-sectional view of a multi-filar conductor bundle 230 in accordance with at least one embodiment of the present disclosure. In the example shown in Figure 13, the multi-filar conductor bundle 230 is a quadrufilar that includes two conductive filars 310 and 330, surrounded by insulating sheaths 315 and 335, respectively. In the example shown in Figure 13, the center two filars of the bundle 230 are reinforcement filars 420, each of which may or may not include an insulating sheath 425. The multi-filar conductor bundle 230 may or may not include an additional overcoating 340 that joins the filars together to form a single conductor bundle 230.

Accordingly, it can be seen that the reinforced multi-filar conductor bundle advantageously increases the strength of multi-filar conductor bundles that may be used in the manufacture of small electronic devices such as intravascular medical catheters and guidewires. A number of variations are possible on the examples and embodiments described above. For example, multiple reinforcing filars, of the same or different types, may be included in a multi-filar conductor bundle, or all of the filars in a multi-filar conductor bundle or cable may be reinforcement type filars.

The logical operations making up the embodiments of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use electrical and electronic devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the reinforced multi-filar conductor bundle. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the reinforced multi-filar conductor bundle as defined in the claims.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

## Claims

1. An intraluminal sensing guidewire, comprising:
a flexible elongate member (106) configured to be positioned within a body lumen of a patient, wherein the flexible elongate member comprises a metallic core wire extending along a longitudinal axis;
a sensing element (112) disposed at a distal portion of the flexible elongate member and configured to obtain physiological data while positioned within the body lumen;
an electrical connector (314) disposed at a proximal portion of the flexible elongate member; and
a filar bundle (230) disposed between the sensing element and the electrical connector, the filar bundle comprising:
a first conductive filar (310; 330) comprising a first material strength, wherein the first conductive filar is in electrical communication with sensing element and the electrical connector;
**characterized in that** the filar bundle further comprises a first reinforcement filar (420) comprising a second material strength greater than the first material strength.

2. The intraluminal sensing guidewire of claim 1, wherein the first conductive filar and the first reinforcement filar extend alongside one another in a helical manner around the metallic core wire.

3. The intraluminal sensing guidewire of claim 1, wherein the filar bundle further comprises a second conductive filar comprising the first material strength, wherein the second conductive filar is in electrical communication with sensing element and the electrical connector.

4. The intraluminal sensing guidewire of claim 1, wherein the filar bundle further comprises a second reinforcement filar comprising the second material strength.

5. The intraluminal sensing guidewire of claim 1, wherein the first reinforcement filar and/or the first conductive filar comprises an insulative coating.

6. The intravascular sensing guidewire of claim 3, wherein the sensing element is a flow sensor, and wherein the first conductive filar, the second conductive filar, and the reinforcement filar extend alongside one another in a helical manner around at least a portion of a length of the metallic core wire.

7. The intraluminal sensing guidewire of claim 1, wherein the filar bundle further comprises an outer coating that mechanically couples the first conductive filar to the first reinforcement filar along at least a portion of a length of the first conductive filar and the first reinforcement filar.

8. The intraluminal sensing guidewire of claim 1, wherein the first conductive filar comprises copper and/or the first reinforcement filar comprises stainless steel.

9. The intraluminal sensing guidewire of claim 1, wherein the first reinforcement filar comprises a cross-sectional diameter larger than a cross-sectional diameter of the first conductive filar.

10. The intraluminal sensing guidewire of claim 1, wherein a length of the first reinforcement filar is less than a length of the first conductive filar.

11. The intraluminal sensing guidewire of claim 1, wherein the filar bundle comprises a helical section and a non-helical section, wherein, in the non-helical section, the first conductive filar extends longitudinally along a length of the flexible elongate member toward at least one electrical contact, wherein the first conductive filar is electrically connected to a conductive ribbon embedded within the flexible elongate member via the electrical contact.

12. A method for assembling an intraluminal sensing guidewire according to any of claims 1-11, comprising:
providing a flexible elongate member configured to be positioned within a body lumen of a patient, wherein the flexible elongate member comprises a metallic core wire extending along a longitudinal axis;
attaching a sensing element at a distal portion of the flexible elongate member, wherein the sensing element is configured to obtain physiological data while positioned within the body lumen;
attaching an electrical connector at a proximal portion of the flexible elongate member;
assembling a filar bundle, wherein assembling a filar bundle comprises coupling a conductive filar to a reinforcement filar, wherein the conductive filar comprises a first material strength, and wherein the reinforcement filar comprises a second material strength greater than the first material strength;
wrapping the filar bundle in a helical manner around the metallic core wire; and
coupling at least the conductive filar of the filar bundle to the sensing element and the electrical connector.

13. The method of claim 12, wherein assembling the filar bundle comprises attaching the conductive filar to the reinforcement filar.

14. The method of claim 13, wherein attaching the conductive filar to the reinforcement filar comprises either attaching an insulating coating of the conductive filar to an insulating coating of the conductive filar or forming an insulating layer around the conductive filar and the reinforcement filar.

15. The method of claim 12, wherein wrapping the filar bundle in the helical manner around the metallic core wire comprises wrapping the filar bundle in the helical manner around a first section of the metallic core wire, and wherein the method further comprises coupling the filar bundle to the flexible elongate member such that the filar bundle extends along a different second section of the metallic core wire in a linear manner.

## Patentansprüche

1. Intraluminaler Sensorführungsdraht, umfassend: ein flexibles, längliches Element (106), das konfiguriert ist, um in einem Körperlumen eines Patienten positioniert zu werden, wobei das flexible, längliche Element einen Metallkerndraht umfasst, der sich entlang einer Längsachse erstreckt; ein Sensorelement (112), das an einem distalen Abschnitt des flexiblen länglichen Elements angeordnet und konfiguriert ist, um physiologische Daten zu erlangen, während es im Körperlumen positioniert ist; einen elektrischen Verbinder (314), der an einem proximalen Abschnitt des flexiblen länglichen Elements angeordnet ist; und ein Fadenbündel (230), das zwischen dem Sensorelement und dem elektrischen Verbinder angeordnet ist, das Fadenbündel umfassend: einen ersten leitenden Faden (310;330), der eine erste Materialstärke aufweist, wobei der erste leitende Faden in elektrischer Verbindung mit dem Sensorelement und dem elektrischen Verbinder ist; **dadurch gekennzeichnet, dass** das Fadenbündel ferner einen ersten Verstärkungsfaden (420) umfasst, der eine zweite Materialstärke aufweist, die größer ist als die erste Materialstärke.

2. Intraluminaler Sensorführungsdraht nach Anspruch 1, wobei sich der erste leitende Faden und der erste Verstärkungsfaden nebeneinander schraubenförmig um den Metallkerndraht erstrecken.

3. Intraluminaler Sensorführungsdraht nach Anspruch 1, wobei das Fadenbündel ferner einen zweiten leitenden Faden umfasst, der die erste Materialstärke aufweist, wobei der zweite leitende Faden in elektrischer Verbindung mit dem Sensorelement und dem elektrischen Verbinder ist.

4. Intraluminaler Sensorführungsdraht nach Anspruch 1, wobei das Fadenbündel ferner einen zweiten Verstärkungsfaden umfassend die zweite Materialstärke umfasst.

5. Intraluminaler Sensorführungsdraht nach Anspruch 1, wobei der erste Verstärkungsfaden und/oder der erste leitende Faden eine isolierende Beschichtung umfasst.

6. Intravaskulärer Sensorführungsdraht nach Anspruch 3, wobei das Sensorelement ein Durchflusssensor ist und wobei sich der erste leitende Faden, der zweite leitende Faden und der Verstärkungsfaden nebeneinander schraubenförmig um mindestens einen Abschnitt der Länge des Metallkerndrahts erstrecken.

7. Intraluminaler Sensorführungsdraht nach Anspruch 1, wobei das Fadenbündel ferner eine äußere Beschichtung umfasst, die den ersten leitenden Faden mit dem ersten Verstärkungsfaden entlang mindestens eines Abschnitts der Länge des ersten leitenden Fadens und des ersten Verstärkungsfadens mechanisch koppelt.

8. Intraluminaler Sensorführungsdraht nach Anspruch 1, wobei der erste leitende Faden Kupfer umfasst und/oder der erste Verstärkungsfaden Edelstahl umfasst.

9. Intraluminaler Sensorführungsdraht nach Anspruch 1, wobei der erste Verstärkungsfaden einen Querschnittsdurchmesser aufweist, der größer ist als der Querschnittsdurchmesser des ersten leitenden Fadens.

10. Intraluminaler Sensorführungsdraht nach Anspruch 1, wobei die Länge des ersten Verstärkungsfadens geringer ist als die Länge des ersten leitenden Fadens.

11. Intraluminaler Sensorführungsdraht nach Anspruch 1, wobei das Fadenbündel einen schraubenförmigen Abschnitt und einen nicht-schraubenförmigen Abschnitt umfasst, wobei sich der erste leitende Faden in dem nicht-schraubenförmigen Abschnitt in Längsrichtung entlang einer Länge des flexiblen länglichen Elements in Richtung mindestens eines elektrischen Kontakts erstreckt, wobei der erste leitende Faden über den elektrischen Kontakt elektrisch mit einem in das flexible längliche Element eingebetteten leitenden Band verbunden ist.

12. Verfahren zum Zusammensetzen eines intraluminalen Sensorführungsdrahts nach einem der Ansprüche 1-11, umfassend: Bereitstellen eines flexiblen, länglichen Elements, das konfiguriert ist, um in einem Körperlumen eines Patienten positioniert zu werden, wobei das flexible, längliche Element einen Metallkerndraht umfasst, der sich entlang einer Längsachse erstreckt; anbringen eines Sensorelements an einem distalen Abschnitt des flexiblen länglichen Elements, wobei das Sensorelement konfiguriert ist, um physiologische Daten zu erlangen, während es im Körperlumen positioniert ist; Anbringen eines elektrischen Steckers an einem proximalen Abschnitt des flexiblen länglichen Elements; Zusammensetzen eines Fadenbündels, wobei das Zusammensetzen eines Fadenbündels ein Koppeln eines leitenden Fadens mit einem Verstärkungsfaden umfasst, wobei der leitende Faden eine erste Materialstärke umfasst und wobei der Verstärkungsfaden eine zweite Materialstärke umfasst, die größer ist als die erste Materialstärke; Wickeln des Fadenbündels auf schraubenförmige Weise um den Metallkerndraht; und Koppeln mindestens des leitenden Fadens des Fadenbündels mit dem Sensorelement und dem elektrischen Verbinder.

13. Verfahren nach Anspruch 12, wobei das Zusammensetzen des Fadenbündels ein Anbringen des leitenden Fadens an dem Verstärkungsfaden umfasst.

14. Verfahren nach Anspruch 13, wobei ein Anbringen des leitenden Fadens an dem Verstärkungsfaden entweder ein Anbringen einer isolierenden Beschichtung des leitenden Fadens an einer isolierenden Beschichtung des leitenden Fadens oder ein Bilden einer isolierenden Schicht um den leitenden Faden und den Verstärkungsfaden umfasst.

15. Verfahren nach Anspruch 12, wobei das schraubenförmige Wickeln des Fadenbündels auf schraubenförmige Weise um den Metallkerndraht ein schraubenförmiges Wickeln des Fadenbündels auf schraubenförmige Weise um einen ersten Abschnitt des Metallkerndrahts umfasst, und wobei das Verfahren ferner ein Koppeln des Fadenbündels mit dem flexiblen länglichen Element umfasst, sodass sich das Fadenbündel entlang eines anderen zweiten Abschnitts des Metallkerndrahts auf lineare Weise erstreckt.

## Revendications

1. Fil de guidage de détection intraluminal, comprenant:
un élément allongé flexible (106) configuré pour être positionné à l'intérieur d'une lumière corporelle d'un patient, dans lequel l'élément allongé flexible comprend un fil central métallique s'étendant le long d'un axe longitudinal; un élément de détection (112) disposé au niveau d'une partie distale de l'élément allongé flexible et configuré pour obtenir des données physiologiques lorsqu'il est positionné à l'intérieur de la lumière corporelle; un connecteur électrique (314) disposé au niveau d'une partie proximale de l'élément allongé flexible; et un faisceau de fils (230) disposé entre l'élément de détection et le connecteur électrique, le faisceau de fils comprenant: un premier fil conducteur (310;330) comprenant une première résistance de matériau, dans lequel le premier fil conducteur est en communication électrique avec l'élément de détection et le connecteur électrique; **caractérisé en ce que** le faisceau filaire comprend en outre un premier fil de renforcement (420) comprenant une seconde résistance de matériau supérieure à la première résistance de matériau.

2. Fil de guidage de détection intraluminale selon la revendication 1, dans lequel le premier fil conducteur et le premier fil de renfort s'étendent l'un à côté de l'autre de manière hélicoïdale autour du fil d'âme métallique.

3. Fil de guidage de détection intraluminale selon la revendication 1, dans lequel le faisceau de filaments comprend en outre un deuxième filament conducteur comprenant la première résistance de matériau, dans lequel le deuxième filament conducteur est en communication électrique avec l'élément de détection et le connecteur électrique.

4. Fil de guidage de détection intraluminale selon la revendication 1, dans lequel le faisceau filaire comprend en outre un second fil de renforcement comprenant la seconde résistance du matériau.

5. Fil de guidage de détection intraluminale selon la revendication 1, dans lequel le premier fil de renfort et/ou le premier fil conducteur comprend un revêtement isolant.

6. Fil de guidage de détection intravasculaire selon la revendication 3, dans lequel l'élément de détection est un capteur de débit, et dans lequel le premier fil conducteur, le deuxième fil conducteur et le fil de renforcement s'étendent les uns à côté des autres de manière hélicoïdale autour d'au moins une partie d'une longueur du fil d'âme métallique.

7. Fil de guidage de détection intraluminale selon la revendication 1, dans lequel le faisceau de filaments comprend en outre un revêtement externe qui couple mécaniquement le premier filament conducteur au premier filament de renforcement sur au moins une partie d'une longueur du premier filament conducteur et du premier filament de renforcement.

8. Fil de guidage de détection intraluminale selon la revendication 1, dans lequel le premier fil conducteur comprend du cuivre et/ou le premier fil de renfort comprend de l'acier inoxydable.

9. Fil de guidage de détection intraluminale selon la revendication 1, dans lequel le premier fil de renforcement comprend un diamètre en coupe supérieur à un diamètre en coupe du premier fil conducteur.

10. Fil de guidage de détection intraluminale selon la revendication 1, dans lequel une longueur du premier fil de renfort est inférieure à une longueur du premier fil conducteur.

11. Fil de guidage de détection intraluminale selon la revendication 1, dans lequel le faisceau filaire comprend une section hélicoïdale et une section non hélicoïdale, dans lequel, dans la section non hélicoïdale, le premier fil conducteur s'étend longitudinalement le long d'une longueur de l'élément allongé flexible vers au moins au moins un contact électrique, dans lequel le premier fil conducteur est connecté électriquement à un ruban conducteur intégré à l'intérieur de l'élément allongé flexible via le contact électrique.

12. Procédé d'assemblage d'un fil de guidage de détection intraluminal selon l'une quelconque des revendications 1 à 11, comprenant: fournir un élément allongé flexible configuré pour être positionné à l'intérieur d'une lumière corporelle d'un patient, dans lequel l'élément allongé flexible comprend un fil central métallique s'étendant le long d'un axe longitudinal; fixer un élément de détection au niveau d'une partie distale de l'élément allongé flexible, l'élément de détection étant configuré pour obtenir des données physiologiques lorsqu'il est positionné à l'intérieur de la lumière corporelle; fixer un connecteur électrique au niveau d'une partie proximale de l'élément allongé flexible; assembler un faisceau filaire, dans lequel l'assemblage d'un faisceau filaire comprend le couplage d'un fil conducteur à un fil de renforcement, le fil conducteur comprenant une première résistance de matériau supérieure à la première résistance de matériau; enrouler le faisceau filaire de manière hélicoïdale autour du fil d'âme métallique; et coupler au moins le fil conducteur du faisceau de fils à l'élément de détection et au connecteur électrique.

13. Procédé selon la revendication 12, dans lequel l'assemblage du faisceau de fils comprend la fixation du fil conducteur au fil de renfort.

14. Procédé selon la revendication 13, dans lequel la fixation du fil conducteur au fil de renfort comprend soit la fixation d'un revêtement isolant du fil conducteur à un revêtement isolant du fil conducteur, soit la formation d'une couche isolante autour du fil conducteur et du fil de renfort.

15. Procédé selon la revendication 12, dans lequel l'enroulement du faisceau filaire de manière hélicoïdale autour du fil d'âme métallique comprend l'enroulement du faisceau filaire de manière hélicoïdale autour d'une première section du fil d'âme métallique, et dans lequel le procédé comprend en outre le couplage du fil filaire faisceau à l'élément allongé flexible de sorte que le faisceau filaire s'étende le long d'une seconde section différente du fil central métallique de manière linéaire.
